(19) Europäisches Patentamt
European Patent Office
Office européen des brevets

(11) Publication number: **0 417 721 B1**

(12) # EUROPEAN PATENT SPECIFICATION

(45) Date of publication of patent specification: **06.09.95**

(51) Int. Cl.$^{6}$: **C07K 5/027**, C07C 237/22, A61K 38/55, C07C 233/87

(21) Application number: **90117461.5**

(22) Date of filing: **11.09.90**

The file contains technical information submitted after the application was filed and not included in this specification

(54) **Novel peptidase and isomerase inhibitors.**

(30) Priority: **11.09.89 US 405491**

(43) Date of publication of application: **20.03.91 Bulletin 91/12**

(45) Publication of the grant of the patent: **06.09.95 Bulletin 95/36**

(84) Designated Contracting States: **AT BE CH DE DK ES FR GB GR IT LI LU NL SE**

(56) References cited:

**J.A.C.S. III, 371 (1989)**

**Chem. Rev. 75, 177 (1975)**

(73) Proprietor: **MERRELL DOW PHARMACEUTICALS INC.**
**2110 East Galbraith Road**
**Cincinnati**
**Ohio 45215 (US)**

(72) Inventor: **Flynn, Gary A.**
**7121 Euclid Road**
**Cincinnati,**
**Ohio 45243 (US)**
Inventor: **Bey, Philippe**
**7875 Ivygate Lane**
**Cincinnati,**
**Ohio 45242 (US)**

(74) Representative: **VOSSIUS & PARTNER**
**Postfach 86 07 67**
**D-81634 München (DE)**

**EP 0 417 721 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to protease enzyme inhibitors useful for a variety of physiological end-use applications.

In EPA195212 protease inhibitors are disclosed comprising amino acid or peptide derivatives in which the scissile amide bond has been replaced by an electrophilic ketone containing group.

In its broad aspects, this invention relates to analogs of peptidase substrates in which the terminal carboxy group has been replaced by a tricarbonyl (-C(O)C(O)C(O)R) group. These peptidase substrate analogs provide specific enzyme inhibitors for a variety of proteases, the inhibition of which exert valuable pharmacological activities and therefore have useful physiological consequences in a variety of disease states.

In its more specific aspects, this invention relates to triketocarbonyl analogs of certain peptidase substrates which are useful in inhibiting serine-, carboxylic acid-, thiol-, and metallo-proteinases, the inhibition of which will have useful physiological consequences in a variety of disease states.

Still more specifically, this invention relates to tri keto analogs of peptidase substrates which fall within the following generic groupings characterized according to their active site dependencies. Such generic groupings are:

I. Serine Proteinases: These include such enzymes such as Elastase (human leukocyte), Cathepsin G, Thrombin, Plasmin, C-1 Esterase, C-3 Convertase, Urokinase, Plasminogen Activator, Acrosin, β-Lactamase, D-Alanine-D-Alanine Carboxypeptidase, Chymotrypsin, Trypsin and Kallikreins.

II. Carboxylic Acid Proteinases: These include such specific enzymes as Renin, Pepsin and Cathepsin D.

III. Metallo Proteinases: These include Angiotensin Converting Enzyme, Enkephalinase, Pseudomonas Elastase and Leucine Aminopeptidase.

IV. Thiol Proteinases: Cathepsin B and Calpain.

The contemplated peptidase inhibitors of the foregoing enzymes are selected from the generic formula

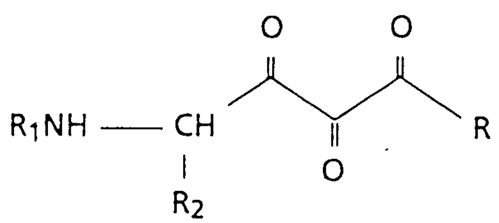

I

the hydrates, or the pharmaceutically acceptable salts thereof wherein:

- R is $-OR^1$ or $-NR^2R^3$ wherein, $R^1$, $R^2$, and $R^3$ are each independently selected from hydrogen, a $(C_1\text{-}C_6)$alkyl group, $(C_2\text{-}C_6)$alkanoyl, phenyl, benzyl, benzoyl, cyclohexyl, cyclohexylmethyl or 2-pyridylmethyl,
- $R_1$ is $-P_2\text{-}P_3\text{-}P_4\text{-}P_5$ with
- $P_2$ being selected from Groups D, E, G and Ⓚ,
- $P_3$ is selected from Groups E, G and K or is deleted,
- $P_4$ is selected from Groups E, G and K or is deleted,
- $P_5$ is selected from Group K or is deleted, and
- $R_2$ is a side chain of an amino acid selected from group F.

Hydrates of the triketo compounds having structure I' are

I'

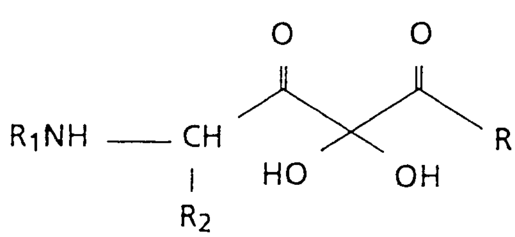

much more chemically stable than are the unhydrated triketo compounds of formula I. For this reason, the hydrates are preferred and any reference in this specification and claims to a triketo (COCOCO) compound should be taken to include reference to the corresponding hydrated form as context allows. Moreover, the compounds of this invention are expected to be in the hydrated form under normal physiological conditions.

Unless otherwise stated, the α-amino acid building blocks of these peptidase substrate analogs are preferably in their L-configuration. As is conventional nomenclature used by peptide chemists, the code for an amino acid wherein the first (or other) letter of the code is upper case indicates that the amino acid has the natural "L" configuration and wherein the first (or other) letter of the code is lower case indicates that the amino acid has "D" configuration. Throughout this specification reference will be made to lower case amino acid codes or codes proceeded by "(D)-" and these shall both be taken as equivalent.

Those compounds of this invention having aspartic or glutamic acid moieties may be in free form or a salt form, e.g., acid addition or anionic salt. Such a compound may be converted into its salt or base form in an art-known manner, one from another. Preferred salts are trifluoroacetate, hydrochloride, sodium, potassium, or ammonium salts, although the scope of salts embraced herein is not limited thereto, the scope being extended to include all of the salts known to be used in the art of peptide chemistry.

Before further defining and/or illustrating the scope of the peptidase inhibitors embraced by formula I, it may be convenient to state some of the more basic concepts related to peptides. Each α-amino acid has a characteristic "R-group", the R-group being the side chain, or residue, attached to the α-carbon atom of the α-amino acid. For example, the R-group side chain for glycine is hydrogen, for alanine it is methyl, for valine it is isopropyl. (Thus, throughout this specification the $R_2$ moiety is the R-group for each indicated α-amino acid). For the specific R-groups - or side chains - of the α-amino acids reference to A.L. Lehninger's text on Biochemistry (see particularly Chapter 4) is helpful.

As a further convenience for defining the scope of the compounds embraced by the generic concept of formula I, as well as the sub-generic concepts relating to each of the individual enzymes involved in this invention, various α-amino acids have been classified into a variety of groups which impart similar functional characteristics for each of the specific enzymes to be inhibited by the peptidase substrates of formula I. These groups are set forth in Table II and the recognized abbreviations for the α-amino acid blocks are set forth in Table I.

| TABLE I | |
|---|---|
| **AMINO ACID** | **SYMBOL** |
| Alanine | Ala |
| Arginine | Arg |
| Aspargine | Asn |
| Aspartic acid | Asp |
| Asn + Asp | Asx |
| Cysteine | Cys |
| Glutamine | Gln |
| Glutamic acid | Glu |
| Gln + Glu | Glx |
| Glycine | Gly |
| Histidine | His |
| Isoleucine | Ile |
| Leucine | Leu |
| Lysine | Lys |
| Methionine | Met |
| Phenylalanine | Phe |
| p-Guanidinophenylalanine | Phe(Gua) |
| Proline | Pro |
| Serine | Ser |
| Threonine | Thr |
| Tryptophan | Trp |
| Tyrosine | Tyr |
| Valine | Val |
| Norvaline | Nva |
| Norleucine | Nle |
| 1-Naphthylalanine | Nal(1) |
| 2-Indolinecarboxylic acid | Ind |

| TABLE I | |
|---|---|
| AMINO ACID | SYMBOL |
| Sarcosine | Sar |
| Cyclohexylalanine | Cha |
| beta-Alanine | bAla |
| beta-Valine | bVal |
| O-4'-Methyltyrosine | Tyr(Me) |
| 3-Pyrazolylalanine | Ala(3pyr) |
| 4-Pyrimidinylalanine | Ala(4pyr) |
| $N^6$-(2-carboxybenzoyl)lysine | Lys(2CBz) |
| Terephthaloyl | tPht |
| $N^6$-acetyllysine | Lys(Ac) |

TABLE II

Group

D: Pro, Ind

E: Ala, bAla, Leu, Ile, Val, Nva, bVal, Met, Nle and N-methyl derivatives

F: Phe, Tyr, Tyr(Me), Ala(3pyr), Ala(4pyr), Trp, Nal(1), and N-methyl derivatives

G: Gly, Sar

K: Acetyl (Ac), Succinyl (Suc), Benzoyl (Bz), t-Butyloxycarbonyl (Boc), Carbobenzyloxy (Cbz), Tosyl (Ts), Dansyl (Dns), Isovaleryl (Iva), Methoxysuccinyl (MeOSuc), 1-Adamantanesulfonyl ($AdSO_2$), 1-Adamantaneacetyl (AdAc), 2-Carboxybenzoyl (2CBz), Phenylacetyl (PhAc), t-Butylacetyl (Tba), bis[(1-naphthyl)methyl]acetyl (BNMA),

or $-A-R_z$ wherein

A is

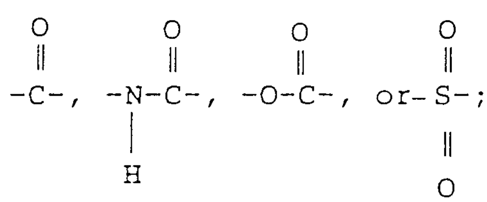

and

$R_z$ is an aryl group containing 6, 10 or 12 carbons suitably substituted by 1 to 3 members selected independently from the group consisting of fluoro, chloro, bromo, iodo, trifluoromethyl, hydroxy, alkyl containing from 1 to 6 carbons, alkoxy containing from 1 to 6 carbons, carboxy, alkylcarbonylamino wherein the alkyl group contains 1 to 6 carbons, 5-tetrazolyl, and acylsulfonamido (i.e., acylaminosulfonyl and sulfonylaminocarbonyl "Sac") containing from 1 to 15 carbons, provided that when the acylsulfonamido contains an aryl the aryl may be further substituted by a member selected from fluoro, chloro, bromo, iodo and nitro; and such other terminal amino protecting groups which are functionally equivalent thereto.

In light of the foregoing, the defined compounds of formula I may also be stated as being

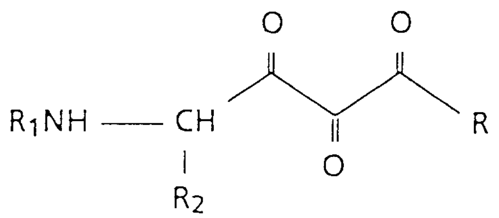

I

the hydrates, or the pharmaceutically acceptable salts thereof wherein $R_1$, $R_2$ and R are as defined above.

The compounds of formula I can also be depicted as a peptide derivative, albeit modified on its carboxy terminal end. In this depiction the $R_2$ moiety is in the $P_1$ position of the peptide, the $\alpha$-amino acids of the $R_1$ moiety would be in the $P_2$-->$P_n$ positions, n being the numeric sequence dependent upon the number of $\alpha$-amino acids in that particular compound, e.g., if $R_1$ contained four $\alpha$-amino acids it would be comprised of $P_2$-$P_3$-$P_4$-$P_5$ positions with the option of a terminal amino protecting group from Group K in the $P_5$ moiety.

To further illustrate the shorthand nomenclature used throughout this application assume that $R_1$ is comprised of $P_2$, $P_3$, $P_4$ having a terminal amino protecting group so that $R_1$ is -Pro-Ala-Ala-MeOSuc, $R_2$ is isopropyl, then that specific compound would be written as MeOSuc-Ala-Ala-Pro-Val.

It is also to be noted that in some instances it is more convenient to designate the terminal amino protecting group as a separate $P_n$ position of the peptide. The terminal amino protecting group would be designated as being in the $P_5$ position and thus $R_1$ would be $P_2$-$P_3$-$P_4$-$P_5$ with $P_5$ being a protecting group of Group K. If $P_4$ optionally is deleted, then quite obviously, when $P_4$ is deleted the protecting group of $P_5$ would be attached to the $P_3$ moiety. In those instances wherein Group K represents an -A-$R_z$ moiety, it is preferred that A represent -C(=O)- and that $R_z$ represent acylsulfonamido, particularly those wherein the acylsulfonamido contains an aryl moiety (preferably phenyl) substituted by a halogen, the preferred -A-$R_z$ moieties being 4-[(4-chlorophenyl)sulfonylaminocarbonyl]phenylcarbonyl, 4-[(4-bromophenyl)-sulfonylaminocarbonyl]phenylcarbonyl and 4-[(4-Iodophenyl)sulfonylaminocarbonyl] phenylcarbonyl (said moieties being abbreviated as ClΦSacBz, BrΦSacBz and IΦSacBz, respectively).

The compounds of formula I which are useful as inhibitors of chymotrypsin are represented by the structural formula Id:

$R_1NHCH(R_2)C(=O)C(=O)C(=O)R$ (Id)

and the hydrates, isosteres or the pharmaceutically acceptable salts thereof, wherein

- R is $-OR^1$ or $-NR^2R^3$ wherein, $R^1$, $R^2$, and $R^3$ are each independently selected from hydrogen, a $(C_1$-$C_6)$alkyl group, $(C_2$-$C_6)$alkanoyl, phenyl, benzyl, benzoyl, cyclohexyl, cyclohexylmethyl or 2-pyridylmethyl with $OR^1$ being preferred especially wherein $R^1$ is a methyl group,
- $R_1$ is $-P_2$-$P_3$-$P_4$-$P_5$ with
- $P_2$ being selected from Groups D and E, with Leu being preferred, or from Groups G and K with benzoyl being preferred,
- $P_3$ is selected from Groups E, G, and K, or is deleted, with alanine and acetyl being preferred,
- $P_4$ is selected from Groups E, G, and K or is deleted, with alanine being preferred, and
- $P_5$ is selected from Group K with succinyl being preferred or is deleted, and
- $R_2$ is selected from the side chains of the amino acids of Group F but preferably is the Phe side chain or the Tyr side chain.

The end-use application of the compounds (Id) inhibiting chymotrypsin is in the treatment of pancreatitis. For their end-use application, the potency and other biochemical parameters of the enzyme inhibiting characteristics of the compounds of (Id) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred. Preferred compounds are as expressed for Cathepsin G and also include:

$Bz$-Phe-$C(O)C(O)OCH_3$,

$Bz$-Tyr-$C(O)C(O)OCH_3$, and

Ac-Leu-Phe-C(O)C(O)$OCH_3$.

Having defined the scope of the compounds within the generic invention and within the individual subgeneric groups for each of the individual enzymes, the manner in which such may be prepared will be described and illustrated.

In general, the compounds of formula I may be prepared using standard chemical reactions analogously known in the art. The procedure for preparing the formula I compounds is outlined in Scheme A wherein R, $R_1$, and $R_2$ are as previously defined. Specifically, the compounds of formula 1 can be prepared by treatment of the appropriate ylide of formula 2 with (a) ozone and dimethyl sulfide or (b) singlet oxygen. The ozonolysis reaction can be conveniently performed by, for example, bubbling an excess of ozone through a cooled solution of the appropriate formula 2 ylide. Suitable solvents include any nonreactive solvent in which the formula 2 ylide is soluble, for example, alkyl esters of simple alkanoic acids such as ethylacetate; the chlorinated hydrocarbons such as carbon tetrachloride, chloroform, 1,2-ethylenedichloride, 1,1,2,2-tetrachloroethane, and methylene chloride; the aromatic hydrocarbons such as benzene, toluene, and xylene; a chlorinated aromatic such as 1,2,4-trichlorobenzene and o-dichlorobenzene; an alcohol such as methanol, ethanol, and isopropanol; or an ethereal solvent such as diethyl ether, tetrahydrofuran (THF), and p-dioxan. Methylene chloride is preferred.

The temperature of the ozonolysis reaction mixture can be any temperature conducive to the reaction, typically from about -78 °C to about 0 °C, preferably from about -78 °C to about -35 °C, and most preferably about -70 °C. The time of the reaction will vary depending on the ylide, the concentration of the reactants, the temperature and other factors. Conveniently, ozone is bubbled into the reaction mixture until the solution turns blue indicating an excess of ozone.

The ozonide is then treated with an excess of a reducing agent such as zinc metal or preferably dimethylsulfide. The desired formula 1 compound as the hydrate is isolated from the reaction mixture in any convenient manner, typically by solvent removal (via evaporation). Purification may be accomplished by, for example, flash chromatography.

Oxidations utilizing singlet oxygen are well known. More specifically, singlet oxygen oxidation of an ylide to produce a tricarbonyl ester has been reported by H. Waserman et al., J. Amer. Chem. Soc. 11, 371 (1989).

Singlet oxygen can be generated by dye-sensitized excitation of oxygen. Suitable dyes include Rose Bengal, Eosin Y and methylene blue. Other sensitizers include dinaphthalenethiophene. Typically Rose Bengal and Eosin Y are attached to a basic anion-exchange resin and methylene blue is attached to an acidic cation-exchange resin. Excitation is accomplished with a UV lamp such as a tungsten-iodine lamp. Suitable solvents are any solvents which promote and do not interfere with the desired reaction. Such solvents include the aromatic hydrocarbons such as benzene and toluene; hydrocarbons such as hexane; ethereal solvents such as diethyl ether, tetrahydrofuran (THF) p-dioxan; chlorinated hydrocarbons such as dichloromethane and chloroform; carbondisulfide; and alcohols such as methanol, ethanol, propanol, isopropanol and t-butyl-alcohol. Mixtures are operable. The temperature of the reaction mixture can be any suitable temperature from about -78 °C to about 30 °C typically from about -78 °C to about -50 °C. The time of the reaction will vary depending on the reactant, the solvent, concentrations, and temperature and can be from about 1 min to about 2 hours. Purification and isolation can be by those methods described above for specification and isolation of product from the ozonolysis reaction mixture.

The formula 2 ylide is prepared from the appropriate N-protected ylide, preferably from the phthaloyl protected ylide of formula 3. The removal of the phthaloyl group can be readily achieved by methods generally known to those skilled in the art. For example, a solution of the phthaloyl ylide can be allowed to react with hydrazine hydrate, typically about a 20-fold excess of hydrazine hydrate, until the reaction is substantially complete. The solvent can be any of those described above for the ozonolysis reaction and preferably will be an alcohol solvent such as EtOH. The temperature of the reaction mixture can be from about 0 °C to about 60 °C, conveniently at about room temperature, i.e., 25 °C. The reaction time will vary depending on the specific reactant, the temperature, the solvent, and other factors known to influence reaction time. Conveniently the progress of the reaction can be monitored by thin layer chromatography (TLC).

Subsequent to removal of the phthaloyl group, the $R_1$ group can be linked to the now free amino group. When $R_1$ is an amino acid, protected amino acid or a peptide comprised of a number of amino acids optionally bearing an amino protecting group, the $R_1$ group can be linked to the unprotected, free amino compound by well known peptide coupling techniques.

In coupling individual amino acids or peptides to the deprotected formula 3 compound, appropriate side chain protecting groups are employed. The selection and use of an appropriate protecting group for these side chain functionalities is within the ability of those skilled in the art and will depend upon the amino acid

to be protected and the presence of other protected amino acid residues in the peptide. The selection of such a side chain protecting group is critical in that it must not be removed during the deprotection and coupling steps of the synthesis. For example, when Boc is used as the α-amino protecting group, the following side chain protecting groups are suitable: p-toluenesulfonyl (tosyl) moieties can be used to protect the amino side chains of amino acids such as Lys and Arg; p-methylbenzyl, acetamidomethyl, benzyl (Bzl), or t-butylsulfonyl moieties can be used to protect the sulfide containing side chains of amino acids such as cysteine, homocysteine, penicillamine and the like or derivatives thereof; benzyl (Bzl) or cyclohexyl ester moieties can be used to protect carboxylic acid side chains of amino acids such as Asp, Glu; a benzyl (Bzl) ether can be used to protect the hydroxy containing side chains of amino acids such as Ser and Thr; and a 2-bromocarbobenzoxy (Z(Br)) moiety can be used to protect the hydroxy containing side chains of amino acids such as Tyr. These side chain protecting groups are added and removed according to standard practices and procedures well known in the art. It is preferred to deprotect these side chain protecting groups with a solution of anisole in anhydrous hydrogen fluoride (1:10). Typically, deprotection of side chain protecting groups is performed after the peptide chain synthesis is complete but these groups can alternatively be removed at any other appropriate time. It is preferred to deprotect these side chains at the same time as the peptide is cleaved from the resin when solid phase synthetic methods are employed.

The phthaloyl (Pht) ylide of formula 3 is prepared by reaction of the phthaloyl protected acid chloride of formula 4 with the phosphonium ylide of formula 5. This reaction is performed by adding a solution of the appropriate formula 5 ylide, preferably dropwise, to a solution of the formula 4 acid chloride. Suitable solvents include those listed above for the ozonolysis reaction and will preferably be an ethereal solvent such as THF. The reaction will require from about 30 minutes to about 12 hours, typically about 2 to 3 hours, depending on the acid chloride, the ylide, the solvent(s), and the temperature which can be from about 0 °C to about 60 °C, conveniently at about room temperature, i.e., 25 °C. Isolation and purification is accomplished by filtering the reaction mixture to remove solid products and subsequently chromatographing the filtrate, for example, on silica gel eluting with a 50% mixture of ethyl acetate and hexane.

The formula 5 phosphonium ylide, Wittig reagent, is prepared from the corresponding formula 6 α-halocarboxylic acid derivative in the usual manner, that is, by reacting the α-halo ester with a tertiary phosphine such as triphenylphosphine to yield a phosphonium salt. When treated with a strong base such as an organolithium compound, for example, lithium diisopropylamide (LDA), sodium hydride, or sodium amide, the acidic proton is removed and the desired ylide is formed. Suitable solvents used in forming the Wittig reagent include any nonreactive solvent, for example, the aromatic hydrocarbons such as benzene or toluene, the chlorinated hydrocarbons such as carbon tetrachloride, chloroform, or methylene chloride, or the ethereal solvents such as diethyl ether or THF.

The reaction can conveniently be performed at from about 0 °C to about 60 °C, typically at room temperature, that is about 25 °C. The halo group of the α-halo ester is preferably a bromo group, but can be a chloro or iodo group or can be any good leaving group which forms a stable phosphonium salt such as a mesylate or tosylate group.

The acid chloride of formula 4 is prepared from the corresponding acid of formula 7 by, for example, reacting the acid with refluxing α,α-dichloromethyl methylether. After about 3 hours, the solution is allowed to cool and the product concentrated by solvent evaporation. The resulting crude acid chloride can be used directly without further purification in the reaction with the formula 5 phosphine.

## Reaction Scheme A

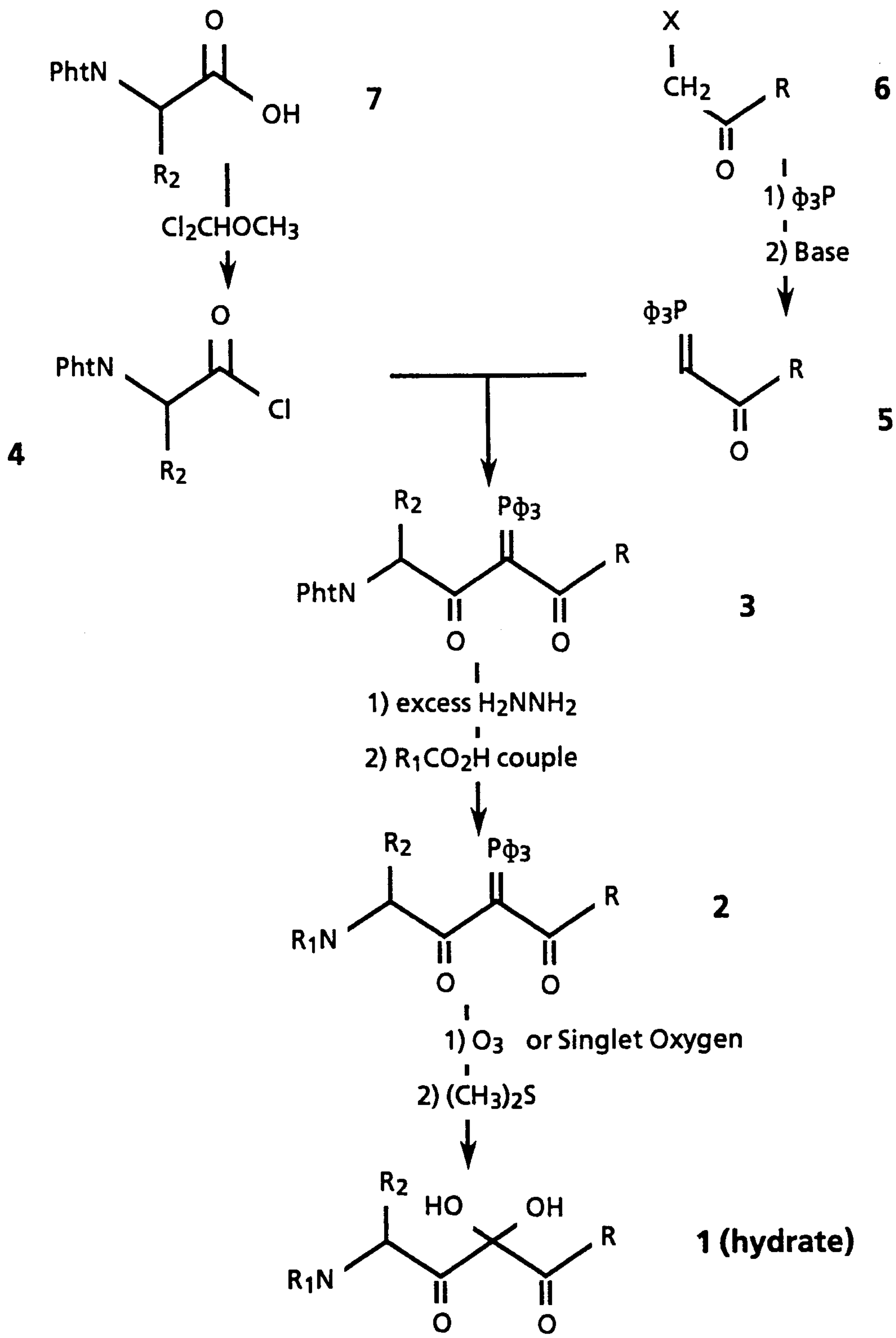


7

6

4

5

3

2

1 (hydrate)

**EXAMPLE 1**

PREPARATION OF (S)-α,α,-DIHYDROXY-β-OXO-γ-[[(PHENYLMETHOXY)CARBONYL]AMINO]-BENZENEPENTANOIC ACID (Cbz-Phe-$C(OH)_2$-$CO_2Et$)

A. Preparation of (Pht-Phe-$C(P\Phi_3)$-$CO_2Et$)

Procedure: To a 25 ml round-bottomed flask under argon atmosphere was added 800 mg (2.76 mmol) of N-phthaloyl-L-phenylalanine and 4 ml of α,α-dichloromethyl methyl ether. The solution was heated at reflux for 3 hours. The cooled solution was concentrated to give 850 mg of a solid. This crude acid chloride was dissolved in 5 ml of dry THF and a solution of 1.74 g (5.0 mmol) of carbethoxymethylidenetriphenylphosphorane in 5 ml THF over 5 min. at 25 °C. A precipitate formed. After stirring for 2 hours, the mixture was filtered and the filtrate chromatographed in 150 g silica gel with 50% EtOAc/hexane. The desired fractions were combined to yield 1.31 g (2.09 mmol) of a colorless foam (76% yield). $^1$H-NMR ($CDCl_3$) δ 7.5-7.7 (m,8H), 7.3-7.5 (m,17H). 7.12 (t,2H,J = 6Hz), 7.03 (t,1H,J = 6Hz), 6.28 (dd,1H, Ja = 10Hz, Jb = 4 Hz), 3.90 (m,2H), 3.75 (m,2H), 0.60 (t,3H,J = 7Hz).

B. Preparation of (S)-β-oxo-γ-[[(phenylmethoxy)carbonyl]-amino]-α-(triphenylphosphoranylidene)-benzenepentanoic acid ethyl ester (Cbz-Phe-$C(P\Phi_3)CO_2Et$)

A solution of 313 mg (0.50 mmol) phthaloyl ylide from Part A in 3 ml anhydrous ethyl alcohol was treated with 500 mg (10 mmol) hydrazine hydrate at 25 °C under nitrogen atmosphere. After 1 hour, no starting material was detected by TLC analysis. The volatiles were removed *in vacuo*. The residue was triturated with $CHCl_3$ and filtered. The filtrate was washed repeatedly with $H_20$, dried over $MgSO_4$, and concentrated to a foam. The crude amine was dissolved in 3 ml $CH_2Cl_2$ and 10 drops of pyridine. A solution of 90 mg (0.5 mmol) benzyl chloroformate in 1 ml $CH_2Cl_2$ was added. After stirring for 30 minutes at 25 °C, the solution was poured into $H_2O$ and extracted with $CH_2Cl_2$. The organic extract was washed well with $H_20$, dried over $MgSO_4$, concentrated, and flash chromatographed to give 245 mg (0.39 mmol, 78% yield) of a colorless glass. $^1$H-NMR ($CDCl_3$) δ 7.4-7.8 (m,15H), 7.15-7.35 (m,10H), 5.87 (m,1H), 5.61 (d,1H,J = 7 Hz), 5.03 (d,1H,J = 12 Hz), 4.96 (d,1H,J = 12Hz), 3.7-3.9 (m,2H), 3.41 (dd,1H,Ja = 14 Hz,Jb = 4 Hz), 2.85 (dd,1H,Ja = 14 Hz, Jb = 8 Hz), 0.72 (t,3Hz,J = 7 Hz).

C. Preparation of (S)-α,α-dihydroxy-β-oxo-γ-[[(phenylmethoxy)carbonyl]amino]benzenepentanoic acid (Cbz-Phe-$C(OH)_2$-$CO_2Et$)

A solution of 100 mg of stabilized ylide from Part B was dissolved in 25 ml $CH_2Cl_2$ at -70 °C and a stream of ozone was bubbled into the solution until it turned blue. 0.5 ml dimethyl sulfide was added. The solution was evaporated and flash chromatographed using 30% ethyl acetate in hexane to give 17 mg of a foam. $^1$H-NMR ($CDCl_3$) δ 7.1-7.4 (m,10H), 5.20 (bs,1H), 5.16 (d,1H,J = 7Hz), 5.05 (m,1H), 5.01 (s,2H), 4.16 (m,2H), 3.25 (dd,1H,Ja = 14 Hz,Jb = 5 Hz), 2.96 (dd,1H,Ja = 14 Hz,Jb = 8 Hz), 1.75 (bs,1H), 1.22 (t,3H,J = 7 Hz).

**EXAMPLE 2**

PREPARATION OF Cbz-Phe-$C(OH)_2$-$C(O)N(CH_3)CH_2C_6H_5$

A. Preparation of $ClCH_2C(O)N(CH_3)CH_2C_6H_5$

To a stirred solution of 8 ml (0.1 mole) chloroacetyl chloride in 100 ml $CH_2Cl_2$ at 0 °C was added 25.7 mL (0.2 mol) benzylmethylamine via dropping funnel over 10 min. The mixture was stirred for 30 min., poured into $H_2O$, extracted into $CH_2Cl_2$, and dried over $MgSO_4$. Concentration gave 19.0 g (0.095 mole) of amide as a clear liquid.

B. Preparation of $\Phi_3P$-$CH_2C(O)N(CH_3)CH_2C_6H_5$

To a stirred solution of 4.0 g (20 mmol) of crude amide chloride from 2A above in 15 mL dry THF was added 5.24 g (20 mmol) $Ph_3P$ and 500 mg NaI. The solution was stirred at 25 °C for 18 hours. Cooling and dilution with hexane and filtration gave 6.85 g of a white hygroscopic powder. Material loss was due to

hygroscopic nature of the compounds.

C. Preparation of PhtN-Phe-C(=PΦ$_3$)C(O)N($CH_3$)$CH_2C_6H_5$

1.6 g (3.5 mmol) of phosphonium salt from 2B was stirred in 25 mL $H_2O$ and treated with 420 mg (7 mmol) of n-butyllithium. 1.15 g (3.5 mmol) acid chloride of PhtN-Phe in 10 mL $CH_2Cl_2$ was added and the mixture was stirred vigorously for 15 min. TLC showed one major spot. The $CH_2Cl_2$ layer was removed, concentrated and flash chromatographed using 70% EtOAc/Hexane on 150 mL silica.
Chromatographed yield was 1.75 g (ca. 2.5 mmol), 71% yield.

D. Preparation of Cbz-Phe-C(=PΦ$_3$)-C(O)N($CH_3$)$CH_2C_6H_5$

1.45 g (ca 2.0 mmol) of phthalimide ylide from 2C was dissolved in 20 mL methanol and treated with 1.0 g (20 mmol) of hydrazine hydrate at 15°C for 45 minutes. The solution was concentrated *in vacuo* at 20°C to a gum. This material was taken up in $CHCl_3$, washed with $H_2O$, and stirred at 25°C until phthalhydrazide formation was complete. After 5 hours,the $CHCl_3$ was removed *in vacuo*. The residue was taken up in 25 ml methanol and heated at 50°C for 8 hours. At this time TLC showed cleavage complete with very little lactam formation. The mixture was concentrated, triturated with $CHCl_3$ and filtered. Concentration gave the crude amine as an oil which was dissolved in 10 mL $CH_2Cl_2$.
Cbz Coupling - Half of the crude amine in 5 mL $CH_2CL_2$ (~1.0 mmol) was stirred with 0.5 mL pyridine and 155 mL of benzylchloroformate at 25°C. After stirring for 30 minutes, the solution was diluted with 50 mL $CH_2Cl_2$, washed with 5% HCl solution, dried over $MgSO_4$ and concentrated to give 800 mg of a residue. Flash chromatography on 100 mL silica using 50→75% EtOAc/Hexane gave 550 mg of a light yellow foam. Yield is ~78%

E. Preparation of Cbz-Phe-C(OH)$_2$-C(O)N($CH_3$)$CH_2C_6H_5$

220 mg (0.31 mmol) of ylide from 2D was dissolved in 8 mL $CH_2Cl_2$, cooled to -70°C, and treated with $O_3$ until a faint green color formed. 0.5 mL $Me_2S$ was added and the yellow solution was concentrated *in vacuo*. Flash chromatography on 50 g silica using 25→30% EtOAc/Hexane yielded the title compound.

The foregoing describes in detail the generic and specific aspects of the scope of the invention as well as the manner of making and using the invention. In addition thereto, although such procedures are known in the art, references setting forth state of the art procedures by which the compounds may be evaluated for their biochemical effects are also included herein.

For example, human elastase is assayed *in vitro* using chromophoric peptides, succinylalanylalanylalanyl-p-nitroanilide (A1), methoxysuccinylalanylalanylprolylvalyl-p-nitroanilide (A2), and others, all of which are available commercially. The assay buffer, pH 8.0, and assay techniques are similar to those described by Lottenberg, et al. (A3, A4). Enzyme is purified from human sputum (A5), although recently it has become commercially available. Kinetic characterization of immediate inhibitors is by means of the Dixon plot (A6), whereas the characterization of slow- and/or tight-binding inhibitors used data analysis techniques reviewed by Williams and Morrison (A7).

Similarly, the other proteases are assayed and effects of inhibitors are assessed *in vitro* by similar spectroscopic techniques: cathepsin G (A2); thrombin (A3); chymotrypsin (A8); trypsin (A9); plasmin (A3); Cl esterase (A10); urokinase (A3); plasminogen activator (A11); acrosin (A12); beta-lactamase (A13); cathepsin B (A14); pepsin (A15); cathepsin D (A16) and leucine aminopeptidase (A17). Pseudomonas elastase was measured in a coupled assay procedure using a human elastase substrate and microsomal aminopeptidase.

Radiometric assays of angiotensin I-converting enzyme and enkephalinase and their inhibitors were based on the procedure of Ryan (A18) and used tritiated substrates purchased from Ventrex Laboratories, Inc. Radioimmunoassay was used for studies with renin (A19). C3-convertase was measured as described by Tack, et al. (A20).

The individual assay references are elaborated upon by the following:

A1. The synthesis and analytical use of a highly sensitive and convenient substrate of elastase. J. Bieth, B. Spiess and C.G. Wermuth, Biochemical Medicine, 11 (1974) 350-375.

A2. Mapping the extended substrate binding site of cathepsin G and human leukocyte elastase. Studies with peptide substrates related to the alpha 1-protease inhibitor reactive site. K. Nakajima, J.C. Powers, B.M. Ashe and M. Zimmerman, The Journal of Biological Chemistry, 254 (1979) 4027-4032.

A3. Assay of coagulation proteases using peptide chromogenic and fluorogenic substrates. R. Lottenberg, U. Christensen, C.M. Jackson and P.L. Coleman, in Methods in Enzymology (L. Lorand, ed),

Academic Press, New York, 1979, vol. 80, pp. 341-361.
A4. Solution composition dependent variation in extinction coefficients for p-nitroaniline. R. Lottenberg and C.M. Jackson, Biochimica et Biophysica Acta, 742 (1983) 558-564.
A5. A rapid procedure for the large scale purification of elastase and cathepsin G from human sputum. R.R. Martodam, R.J. Baugh, D.Y. Twumasi and I.E. Liener, Preparative Biochemistry, 9 (1979) 15-31.
A6. The determination of enzyme inhibitor constants. M. Dixon, The Biochemical Journal, 55 (1953) 170-171.
A7. The kinetics of reversible tight-binding inhibition. J.W. Williams and J.F. Morrison, in Methods in Enzymology (D.L. Purich, ed), Academic Press, New York, 1979, vol. 63, pp. 437-467.
A8. Two convenient spectrophotometric enzyme assays. A biochemistry experiment in kinetics. J.A. Hurlbut, T.N. Ball, H.C. Pound and J.L. Graves, Journal of Chemical Education, 50 (1973) 149-151.
A9. The preparation and properties of two new chromogenic substrates of trypsin. B.F. Erlanger, N. Kokowsky and W. Cohen, Archives of Biochemistry and Biophysics, 95 (1961) 271-278.
A10. The human complement system serine proteases Clr and Cls and their proenzymes. R.B. Sim, in Methods in Enzymology (L. Lorand, ed), Academic Press, New York, 1979, vol. 80, pp. 26-42.
A11. Extrinsic plasminogen activator and urokinase. J.H. Verheijen, C. Kluft, G.T.G. Chang and E. Mullaart, in Methods of Enzymatic Analysis (H.U. Bergmeyer, J. Bergmeyer and M. Grassl, eds), Verlag Chemie, Weinheim, 1984, third edition, vol. 5, pp. 425-433.
A12. Sperm acrosin. W. Mueller-Esterl and H. Fritz, in Methods in Enzymology (L. Lorand, ed), Academic Press, New York, 1979, vol. 80, pp. 621-632.
A13. Novel method for detection of beta-lactamases by using a chromogenic cephalosporin substrate. C.H. O'Callaghan, A. Morris, S.M. Kirby and A.H. Shingler, Antimicrobial Agents and Chemotherapy, 1 (1972) 283-288.
A14. Cathepsin B, cathepsin H, and cathepsin L. A.J. Barrett and H. Kirschke, in Methods in Enzymology (L. Lorand, ed), Academic Press, New York, 1979, vol. 80, pp. 535-561.
A15. Pepsins, gastricsins and their zymogens. A.P. Ryle, in Methods of Enzymatic Analysis (H.U. Bergmeyer, J. Bergmeyer and M. Grassl, eds), Verlag Chemie, Weinheim, 1984, third edition, vol. 5, pp. 223-238.
A16. Cathepsin D, cathepsin E. V. Turk, T. Lah and I. Kregar, in Methods of Enzymatic Analysis (H.U. Bergmeyer, J. Bergmeyer and M. Grassl, eds), Verlag Chemie, Weinheim, 1984, third edition, vol 5, pp. 211-222.
A17. Amino acid arylamidase. J.C.M. Hafkenscheid, in Methods of Enzymatic Analysis (H.U. Bergmeyer, J. Bergmeyer and M. Grassl, eds), Verlag Chemie, Weinheim, 1984, third edition, vol. 5, pp. 11-15.
A18. Angiotensin I converting enzyme (kininase II). J.W. Ryan, in Methods of Enzymatic Analysis (H.U. Bergmeyer, J. Bergmeyer and M. Grassl, eds), Verlag Chemie, Weinheim, 1984, third edition, vol. 5, pp. 20-34.
A19. Renin. T. Inagami and M. Naruse, in Methods of Enzymatic Analysis (H.U. Bergmeyer, J. Bergmeyer and M. Grassl, eds), Verlag Chemie, Weinheim, 1984, third edition, vol. 5, pp. 249-258.
A20. The third, fourth, and fifth components of human complement: isolation and biochemical properties. B.F. Tack, J. Janatova, M.L. Thomas, R.A. Harrison and C.H. Hammer, in Methods in Enzymology (L. Lorand, ed), Academic Press, New York, 1979, vol. 870, pp. 64-101.

By following the techniques referenced above, as well as by utilization of other known techniques, as well as by comparison with compounds known to be useful for treatment of the above-mentioned disease states, it is believed that adequate material is available to enable one of ordinary skill in the art to practice the invention. Of course, in the end-use application of the compounds of this invention, the compounds are preferably formulated into suitable pharmaceutical preparations such as tablets, capsules or elixers, for oral administration or in sterile solutions or suspensions for parenteral administration. The compounds of this invention can be administered to patients (animals and human) in need of such treatment in a dosage range of 5 to 500 mg per patient generally given several times, thus giving a total daily dose of from 5 to 2000 mg per day. As stated above, the dose will vary depending on severity of disease, weight of patient and other factors which a person skilled in the art will recognize.

Typically the compounds described above are formulated into pharmaceutical compositions as discussed below.

About 10 to 500 mg of a compound or mixture of compounds of formula I or a physiologically acceptable salt is compounded with a physiologically acceptable vehicle, carrier, excipient, binder, preservative, stabilizer or flavor, in a unit dosage form as called for by accepted pharmaceutical practice. The amount of active substance in these compositions or preparations is such that a suitable dosage in the range indicated is obtained.

Illustrative of the adjuvants which may be incorporated in tablets, capsules and the like are the following: a binder such as gum tragacanth, acacia, corn starch or gelatin; an excipient such as microcrystalline cellulose; a disintegrating agent such as corn starch, pregelatinized starch or alginic acid a lubricant such as magnesium stearate; a sweetening agent such as sucrose, lactose or Saccharin; a flavoring agent such as peppermint, oil of wintergreen or cherry. When the dosage unit form is a capsule, it may contain in addition to materials of the above type, a liquid carrier such as fatty oil. Various other materials may be present as coatings or to otherwise modify the physical form of the dosage unit. For instance, tablets may be coated with shellac, sugar or both. A syrup or elixer may contain the active compound, sucrose as a sweetening agent, methyl and propyl parabens® as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil or cottonseed oil, or a synthetic fatty vehicle like ethyl oleate. Buffers, preservatives or antioxidants can be incorporated as required.

## Claims

**1.** A compound of the formula I:

$R_1NHCH(R_2)C(O)C(O)C(O)R$ (I)

the hydrates, or the pharmaceutically acceptable salts thereof wherein

R is $-OR^1$ or $-NR^2R^3$ wherein $R^1$, $R^2$, and $R^3$ are each independently selected from hydrogen, a $(C_1-C_6)$alkyl group, $(C_2-C_6)$alkanoyl, phenyl, benzyl, benzoyl, cyclohexyl, cyclohexylmethyl or 2-pyridylmethyl,

$R_1$ is $-P_2-P_3-P_4-P_5$ with

$P_2$ being selected from Groups D, E, G and Ⓚ,

$P_3$ is selected from Groups E, G and K or is deleted,

$P_4$ is selected from Groups E, G and K or is deleted,

$P_5$ is selected from Group K or is deleted, and

$R_2$ is a side chain of an amino acid selected from group F; wherein the Groups D, E, F, G and K are defined as follows:

Group

D: Pro, Ind

E: Ala, bAla, Leu, Ile, Val, Nva, bVal, Met, Nle and N-methyl derivatives

F: Phe, Tyr, Tyr(Me), Ala(3pyr), Ala(4pyr), Trp, Nal(1), and N-methyl derivatives

G: Gly, Sar

K: Acetyl (Ac), Succinyl (Suc), Benzoyl (Bz), t-Butyloxycarbonyl (Boc), Carbobenzyloxy (Cbz), Tosyl (Ts), Dansyl (Dns), Isovaleryl (Iva), Methoxysuccinyl (MeOSuc), 1-Adamantanesulfonyl ($AdSO_2$), 1-Adamantaneacetyl (AdAc), 2-Carboxybenzoyl (2CBz), Phenylacetyl (PhAc), t-Butylacetyl (Tba), bis[(1-naphthyl)methyl]acetyl (BNMA),

or $-A-R_z$ wherein

A is

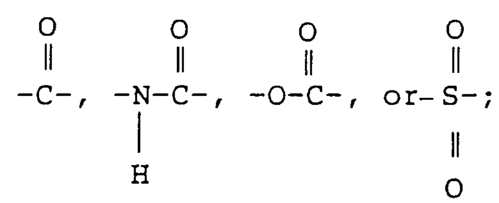

and

$R_z$ is an aryl group containing 6, 10 or 12 carbons suitably substituted by 1 to 3 members selected independently from the group consisting of fluoro, chloro, bromo, iodo, trifluoromethyl, hydroxy, alkyl containing from 1 to 6 carbons, alkoxy containing from 1 to 6 carbons, carboxy, alkylcarbonylamino wherein the alkyl group contains 1 to 6 carbons, 5-tetrazolyl, and acylsulfonamido (i.e., acylaminosulfonyl and sulfonylaminocarbonyl "Sac")

containing from 1 to 15 carbons, provided that when the acylsulfonamido contains an aryl the aryl may be further substituted by a member selected from fluoro, chloro, bromo, iodo and nitro; and such other terminal amino protecting groups which are functionally equivalent thereto.

**2.** A compound of claim 1 selected from the group consisting of
Bz-Phe-C(O)C(O)$OCH_3$,
Bz-Tyr-C(O)C(O)$OCH_3$, and
Ac-Leu-Phe-C(O)C(O)$OCH_3$.

**3.** A process for preparing a compound of the formula

$R_1NHCH(R_2)C(O)C(O)C(O)R$

wherein R, $R_1$ and $R_2$ are as defined in Claim 1 which comprises treating a solution of a compound of the formula

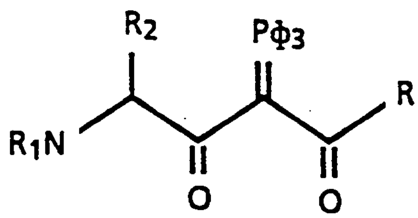

with either a) singlet oxygen or b) excess ozone followed by dimethylsulfide, and isolating the product.

**4.** The compounds of claim 1 or 2 for use as a pharmaceutically active substance.

**5.** The use of the compounds of claim 1 or 2 for the preparation of a pharmaceutical composition to inhibit peptidases and isomerases.

**6.** A method for the preparation of a pharmaceutical composition comprising combining a compound as defined in claim 1 or 2 with a pharmaceutically acceptable carrier.

**7.** A method according to claim 6 wherein the pharmaceutical composition prepared is useful as a peptidase and isomerase inhibitor.

**Patentansprüche**

**1.** Verbindung der Formel I:

$R_1NHCH(R_2)C(O)C(O)C(O)R$, (I)

die Hydrate oder pharmazeutisch verträglichen Salze davon, wobei

- R die Bedeutung $-OR^1$ oder $-NR^2R^3$ hat, wobei $R^1$, $R^2$ und $R^3$ unabhängig voneinander ausgewählt sind aus einem Wasserstoffatom, einem $(C_1-C_6)$Alkylrest, einem $(C_2-C_6)$-Alkanoylrest, einer Phenyl-, Benzyl-, Benzoyl-, Cyclohexyl-, Cyclohexylmethyl- oder 2-Pyridylmethylgruppe;
- $R_1$ die Bedeutung $-P_2-P_3-P_4-P_5$ hat, wobei
- $P_2$ aus den Gruppen D, E, G und K ausgewählt ist,
- $P_3$ aus den Gruppen E, G und K ausgewählt ist oder fehlt,
- $P_4$ aus den Gruppen E, G und K ausgewählt ist oder fehlt,
- $P_5$ aus der Gruppe K ausgewählt ist oder fehlt und
- $R_2$ eine Seitenkette einer Aminosäure ist, ausgewählt aus der Gruppe F, wobei die Gruppen D, E, F, G und K wie folgt definiert sind:
- D: Pro, Ind;

E: Ala, bAla, Leu, Ile, Val, Nva, bVal, Met, Nle und N-Methylderivate;
F: Phe, Tyr Tyr(Me), Ala(3pyr), Ala(4pyr), Trp, Nal(1) und N-Methylderivate;
G: Gly, Sar;
K: Acetyl (Ac), Succinyl (Suc), Benzoyl (Bz), t-Butyloxycarbonyl (Boc), Carbobenzyloxy (Cbz), Tosyl (Ts), Dansyl (Dns), Isovaleryl (Iva), Methoxysuccinyl (MeOSuc), 1-Adamantansulfonyl ($AdSO_2$), 1-Adamantanacetyl (AdAc), 2-Carboxybenzoyl (2CBz), Phenylacetyl (PhAc), t-Butylacetyl (Tba), Bis[(1-naphthyl)methyl]acetyl (BNMA) oder $-A-R_z$, wobei
A die Gruppen

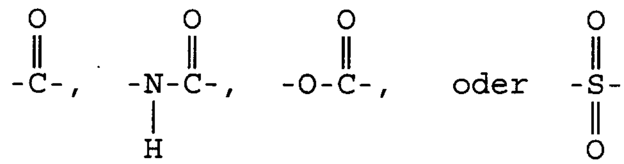
oder

bedeutet, und
$R_z$ ein Arylrest ist, der 6, 10 oder 12 Kohlenstoffatome enthält, geeignet substituiert mit 1 bis 3 Fluor-, Chlor-, Brom- oder Iodatomen, Trifluormethyl- oder Hydroxygruppen, Alkylresten mit 1 bis 6 Kohlenstoffatomen, Alkoxyresten mit 1 bis 6 Kohlenstoffatomen, Carboxylgruppen, Alkylcarbonylaminoresten, wobei der Alkylrest 1 bis 6 Kohlenstoffatome enthält, 5-Tetrazolyl- oder Acylsulfonamidresten (d.h. Acylaminosulfonylresten und Sulfonylaminocarbonylgruppen "Sac") mit 1 bis 15 Kohlenstoffatomen, mit der Maßgabe, daß, falls der Acylsulfonamidrest einen Arylrest enthält, der Arylrest weiter mit Fluor-, Chlor-, Brom- oder Iodatomen oder Nitrogruppen substituiert sein kann; und weitere terminale Aminoschutzgruppen, die funktionelle Äquivalente dazu darstellen.

**2.** Verbindung nach Anspruch 1, ausgewählt aus
$Bz-Phe-C(O)C(O)OCH_3$,
$Bz-Tyr-C(O)C(O)OCH_3$ und
$Ac-Leu-Phe-C(O)C(O)OCH_3$.

**3.** Verfahren zur Herstellung einer Verbindung der Formel

$R_1NHCH(R_2)C(O)C(O)C(O)R$,

in der R, $R_1$ und $R_2$ wie in Anspruch 1 definiert sind, umfassend die Behandlung einer Lösung der Verbindung der Formel

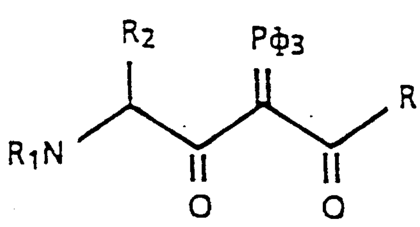

mit entweder a) Singulett-Sauerstoff oder b) Ozon im Überschuß, gefolgt von Dimethylsulfid und Isolierung des Produkts.

**4.** Verbindungen nach Anspruch 1 oder 2 zur Verwendung als pharmazeutisch wirksame Substanz.

**5.** Verwendung der Verbindungen nach Anspruch 1 oder 2 zur Herstellung eines Arzneimittels zur Hemmung von Peptidasen und Isomerasen.

**6.** Verfahren zur Herstellung eines Arzneimittels, umfassend das Kombinieren einer Verbindung nach Anspruch 1 oder 2 mit einem pharmazeutisch verträglichen Träger.

**7.** Verfahren nach Anspruch 6, wobei das hergestellte Arzneimittel als ein Peptidase- und Isomerase-Inhibitor nützlich ist.

## Revendications

**1.** Composé de formule I:

$R_1NHCH(R_2)C(O)C(O)C(O)R$ (I)

ses hydrates ou ses sels pharmaceutiquement acceptables, étant entendu que :

- R est $-OR^1$ ou $-NR^2R^3$, $R^1$, $R^2$ et $R^3$ étant sélectionnés indépendamment parmi l'hydrogène, un groupement alkyle en $C_1$-$C_6$, alcanoyle en $C_2$-$C_6$, phényle, benzyle, benzoyle, cyclohexyle, cyclohexylméthyle ou 2-pyridylméthyle,
- $R_1$ est $-P_2-P_3-P_4-P_5$,
- $P_2$ étant sélectionné dans les groupes D, E, G et K,
- $P_3$ étant sélectionné dans les groupes E, G et K, ou supprimé,
- $P_4$ étant sélectionné dans les groupes E, G et K, ou supprimé,
- $P_5$ étant sélectionné dans le groupe K, ou supprimé et
- $R_2$ est une chaîne latérale d'un aminoacide sélectionné dans le groupe F; étant entendu que les groupes D, E, F, G et K sont définis comme suit :

  Groupe
- D : Pro, Ind
- E : Ala, bAla, Leu, Ile, Val, Nva, bVal, Met, Nle et dérivés N-méthyle
- F : Phe, Tyr, Tyr(Me), Ala(3pyr), Ala(4pyr), Trp, Nal(1) et dérivés N-méthyle
- G : Gly, Sar
- K : acétyle (Ac), succinyle (Suc), benzoyle (Bz), t-butyloxycarbonyle (Boc), carbobenzyloxy (Cbz), tosyle (Ts), dansyle (Dns), isovaléryle (Iva), méthoxysuccinyle (MeOSuc), 1-adamantanesulfonyle ($AdSO_2$), 1-adamantaneacétyle (AdAc), 2-carboxybenzoyle (2CBz), phénylacétyle (PhAc), t-butylacétyle (Tba), bis[(1-naphtyl)méthyl]acétyle (BNMA), ou $-A-R_z$, étant entendu que
- A est

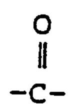, 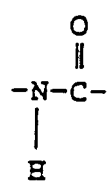, 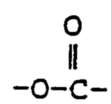, ou 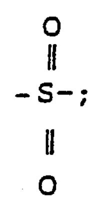;

  et
- $R_z$ est un groupement aryle contenant 6, 10 ou 12 atomes de carbone substitués de façon appropriée par 1 à 3 éléments sélectionnés indépendamment dans le groupe constitué par le fluor, chlore, brome, iode, trifluorométhyle, hydroxyle, alkyle contenant 1 à 6 atomes de carbone, alcoxy contenant 1 à 6 atomes de carbone, carboxyle, alkycarbonylamine, dans lequel le groupement alkyle contient 1 à 6 atomes de carbone, 5-tétrazolyle et acylsulfamide (c'est-à-dire acylaminosulfonyle et sulfonylaminocarbonyle "Sac") contenant 1 à 15 atomes de carbone, pour autant que lorsque l'acylsulfamide contient un groupement aryle, le groupement aryle peut être en outre substitué par un élément sélectionné parmi le fluor, le chlore, le brome, l'iode et l'azote et d'autres groupements aminoprotecteurs terminaux qui sont fonctionnellement équivalents.

**2.** Composé selon la revendication 1, sélectionné dans le groupe constitué par :
$Bz-Phe-C(O)C(O)OCH_3$,
$Bz-Tyr-C(O)C(O)OCH_3$ et
$Ac-Leu-Phe-C(O)C(O)OCH_3$.

**3.** Procédé de préparation d'un composé de formule :

$R_1NHCH(R_2)C(O)C(O)C(O)R$

dans laquelle R, $R_1$ et $R_2$ sont tels que définis à la revendication 1, lequel comprend le traitement d'une solution d'un composé de formule :

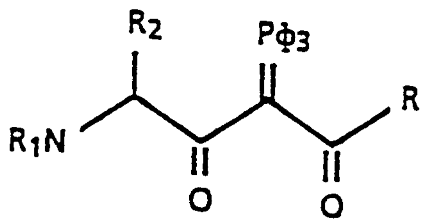

avec a) soit de l'oxygène singulet, b) soit de l'ozone en excès puis du sulfure de diméthyle, et l'isolement du produit.

**4.** Composés selon la revendication 1 ou 2 pour usage comme substance pharmaceutiquement active.

**5.** Utilisation des composés selon la revendication 1 ou 2 pour la préparation d'une composition pharmaceutique destinée à inhiber les peptidases et les isomérases.

**6.** Procédé de préparation d'une composition pharmaceutique comprenant l'association d'un composé selon la revendication 1 ou 2 avec un véhicule pharmaceutiquement acceptable.

**7.** Procédé selon la revendication 6, dans lequel la composition pharmaceutique préparée est utile en tant qu'inhibiteur de peptidases et d'isomérases.